Europäisches Patentamt

European Patent Office

Office européen des brevets

(11) Veröffentlichungsnummer: **0 269 040 B1**

# EUROPÄISCHE PATENTSCHRIFT

(45) Veröffentlichungstag der Patentschrift: **15.04.92**

(21) Anmeldenummer: **87117204.5**

(22) Anmeldetag: **21.11.87**

(51) Int. Cl.⁵: **A45D 29/00**, A61B 17/54

(54) **Hornhautreibe.**

(30) Priorität: **22.11.86 DE 3639953**

(43) Veröffentlichungstag der Anmeldung:
**01.06.88 Patentblatt 88/22**

(45) Bekanntmachung des Hinweises auf die
Patenterteilung:
**15.04.92 Patentblatt 92/16**

(84) Benannte Vertragsstaaten:
**AT BE CH DE ES FR GB GR IT LI LU NL SE**

(56) Entgegenhaltungen:
**DE-B- 2 215 376**
**DE-C- 3 212 274**

(73) Patentinhaber: **Wilkinson Sword Gesellschaft
mit beschränkter Haftung
Schützenstrasse 110
W-5650 Solingen 1(DE)**

(72) Erfinder: **Halm, Hans
Castroper Str. 34
W 4690 Herne1(DE)**

(74) Vertreter: **Patentanwälte Dipl.-Ing. Alex Stenger Dipl.-Ing. Wolfram Watzke Dipl.-Ing.
Heinz J. Ring
Kaiser-Friedrich-Ring 70
W-4000 Düsseldorf 11(DE)**

## Beschreibung

Die Erfindung betrifft eine Hornhautreibe mit einem Handgriff sowie mit einem eine Reibfläche aufweisenden sowie bezüglich zum Handgriff abgewinkelten Kopfteil.

Eine derartige Hornhautreibe für kosmetische Zwecke ist aus der DE-C-32 12 274 bekannt. Sie besteht aus einem Handgriff sowie aus einem vorderen Kopfteil, das mit einer Reibfläche versehen ist, mit der die Hornhaut abgeschabt werden kann. Zur Verbesserung der ergonomischen Eigenschaften und damit für eine einfachere Handhabung der Hornhautreibe bei ihrem Gebrauch ist das Kopfteil mit seiner Reibfläche bezüglich zum Handgriff abgewinkelt.

Bei dieser bekannten Hornhautreibe ist von Nachteil, daß die Reibfläche nur einseitig am Kopfteil angeordnet ist, nämlich an der Außenseite des überstumpfen Winkelbereichs des Kopfteils. Die bekannte Hornhautreibe ist somit nur einseitig benutzbar, und zwar mit einer ganz bestimmten Winkeleinstellung des Handgriffs bezüglich zum Kopfteil. In Abhängigkeit von der jeweils zu behandelnden Körperregion kann aber die Anordnung der Reibfläche an der Außenseite im überstumpfen Winkelbereich des Kopfteils ergonomisch ungünstig sein. Schließlich ist bei der bekannten Hornhautreibe von Nachteil, daß die Geometrie der Reibfläche bezüglich zum Handgriff fest vorgegeben ist.

Davon ausgehend liegt der Erfindung die **Aufgabe** zugrunde, die bekannte Hornhautreibe derart weiterzuentwickeln, daß mit geringem technischen Mehraufwand die ergonomischen Eigenschaften weiter verbessert werden.

Als technische **Lösung** wird mit der Erfindung vorgeschlagen, daß das abgewinkelte Kopfteil drehbar um eine entweder in Längsrichtung des Kopfteils oder in Längsrichtung des Handgriffs verlaufende Achse am Handgriff angeordnet ist.

Eine nach dieser technischen Lehre ausgebildete Hornhautreibe hat den Vorteil, daß mit ein und derselben Reibfläche zwei verschiedene Winkelstellungen der wirksamen Reibfläche bezüglich zum Handgriff möglich sind. So kann die Reibfläche entweder an der Außenseite im überstumpfen Winkelbereich oder aber an der Innenseite im stumpfen Winkelbereich des Kopfteils bezüglich zum Handgriff angeordnet sein, indem diese beiden Positionen einfach durch Drehen des Kopfteils eingestellt werden können. Somit sind die Einsatzmöglichkeiten der Hornhautreibe im Hinblick auf eine weitere Verbesserung der ergonomischen Eigenschaften durch eine einfache konstruktive Maßnahme beträchtlich erweitert worden. Ein weiterer Vorteil der Verdrehbarkeit des die Reibfläche aufweisenden Kopfteils und damit die nichtstarre Anordnung bezüglich zum Handgriff liegt darin, daß sich während der Behandlung die Reibfläche bezüglich der zu behandelnden Körperregion einstellen kann.

Eine Weiterentwicklung der erfindungsgemäßen Hornhautreibe schlägt vor, daß sowohl an der Innenseite (stumpfer Winkelbereich) als auch an der Außenseite (überstumpfer Winkelbereich) des Kopfteils jeweils eine Reibfläche angeordnet ist. Die Hornhautreibe ist somit beidseitig benutzbar, was zum einen die Lebensdauer der Hornhautreibe verlängert. Zum anderen ist es dadurch denkbar, die Hornhautreibe mit zwei unterschiedlichen Reibflächen zu bestücken, um diese dann in die jeweilige Wirkposition zu drehen.

Als Dreheinrichtung kann in einer bevorzugten Weiterbildung ein entsprechend der Abwinklung schräg angeformter Zapfen mit federnden Zungen, die an ihren vorderen Enden Rastnasen aufweisen, sowie eine dazu korrespondierende Ausnehmung, in die der Zapfen einrastet, vorgesehen sein. Durch eine derartige Dreheinrichtung ist nicht nur auf sehr einfache Weise eine Drehmöglichkeit des Kopfteils bezüglich zum Handgriff geschaffen, sondern es ist darüber hinaus noch eine sehr einfache Montage des Kopfteils am Handgriff möglich, indem diese ineinandergesteckt werden, wobei der Zapfen des einen in die Ausnehmung des anderen einrastet. Dies erweist sich insofern als günstig, als beide Teile einstückig hergestellt werden können, ohne daß es zusätzlicher Teile bedarf.

Im einfachsten Fall kann eine Verdrehsicherung zwischen dem Kopfteil und dem Handgriff dadurch gegeben sein, daß beide Teile im Reibungsverbund miteinander stehen. In einer bevorzugten Weiterbildung kann jedoch statt dessen eine Verdrehsicherung zwischen dem Kopfteil und dem Handgriff in Form einer Rasteinrichtung vorgesehen sein, wobei als Rasteinrichtung miteinander korrespondierende Vorsprünge und Ausnehmungen dienen können. Diese Vorsprünge und Ausnehmungen können dabei an den stirnseitigen Berührungsflächen des Kopfteils und des Handgriffs ausgebildet sein, die nach einer Drehung um 180° einrasten.

Vorzugsweise weist das Kopfteil einen ovalen Querschnitt auf, wobei die Reibfläche(n) an der (den) abgeflachten Umfangsfläche(n) angeordnet ist (sind). Auf diese Weise lassen sich sehr großflächige Reibflächen anbringen, die im wesentlichen die gesamte abgeflachte Umfangsfläche des Kopfteils abdecken. Durch die ovale Querschnittsform des Kopfteils ist darüber hinaus die Reibfläche gewölbt ausgebildet und besitzt daher bessere Reibeigenschaften als beispielsweise ebene Reibflächen.

Vorzugsweise ist die Reibfläche als gewölbte Platte ausgebildet. Eine derartige Platte ist einfach in der Herstellung und kann anschließend auf einfache Weise am Kopfteil befestigt werden. Bevorzugt besteht die Reibfläche aus rostfreiem Stahl und ist nach Art eines Schleifbleches oder einer Feile ausgebildet. Ein Schleifblech hält ungefähr zehnmal länger als Sandpapier.

Vorzugsweise ist die Reibfläche von hinten punktförmig geprägt. Dabei kann sie 100 bis 200, vorzugsweise 160 Punkte/cm² aufweisen. Durch diese Prägung wird eine Reibfläche mit sehr guten Reibeigenschaften geschaffen, wobei die Prägepunkte wie Nadelspitzen vorstehen. Insbesondere sind die Abnutzungen nur sehr minimal.

Schließlich wird mit der Erfindung vorgeschlagen, daß die Reibfläche auswechselbar ist. Dies hat den Vorteil, daß eine Hornhautreibe mit verbrauchter Reibfläche nicht weggeworfen werden muß, sondern daß durch den Austausch der Reibfläche gegen eine unverbrauchte die Hornhautreibe so gut wie in den Neuzustand wieder übergeführt wird. Darüber hinaus kann durch die Auswechselmöglichkeit die Reibfläche gegen eine andere mit anderen Reibeigenschaften ausgetauscht werden.

Weitere Einzelheiten und Vorteile ergeben sich aus der nachfolgenden Beschreibung der zugehörigen Zeichnung, in der eine bevorzugte Ausführungsform einer erfindungsgemäßen Hornhautreibe schematisch dargestellt ist. In den Zeichnung zeigt:

Fig. 1 eine Seitenansicht der Hornhautreibe mit abgewinkeltem Kopfteil, teilweise im Schnitt;

Fig. 2 eine Draufsicht auf die Hornhautreibe in fig. 1;

Fig. 3 einen Schnitt entlang der Linie III-III in Fig. 2.

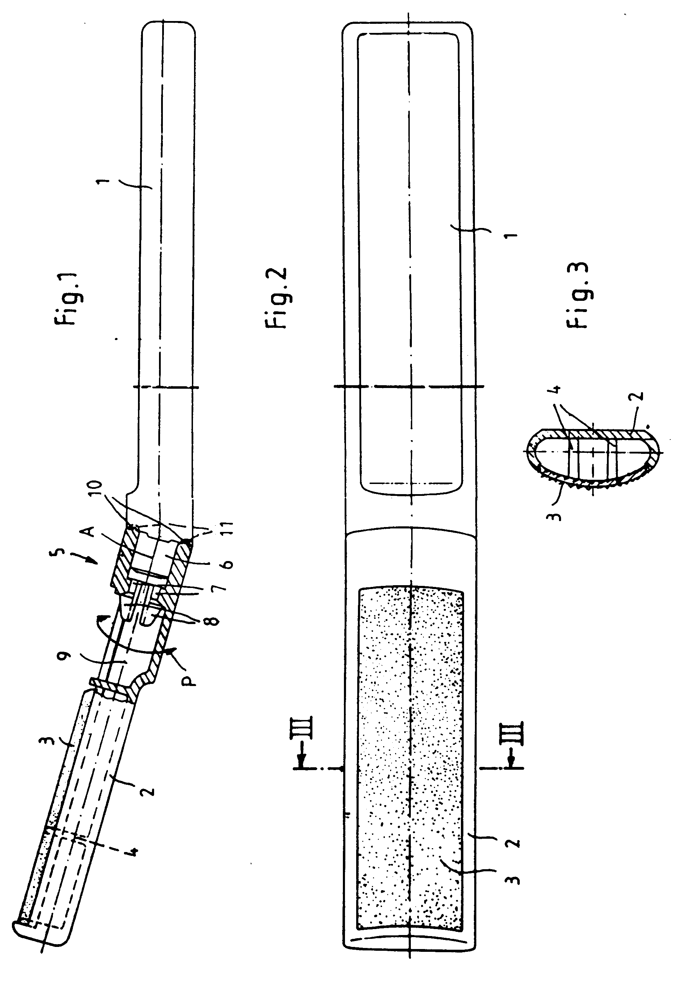

Die dargestellte Hornhautreibe besteht aus einem länglichen Handgriff 1 mit im wesentlichen ovaler Querschnittsform sowie aus einem ebenfalls länglichen Kopfteil 2, das ebenso wie der Handgriff 1 eine im wesentlichen ovale Querschnittsform aufweist, wie in der Schnittdarstellung in Fig. 3 zu erkennen ist. Das Kopfteil 2 weist auf der einen der beiden abgeflachten Umfangsflächen eine im wesentlichen quadratische Reibfläche 3 auf, die als gewölbte Platte ausgebildet ist, wie ebenfalls in Fig. 3 zu erkennen ist. Die Reibfläche 3 stützt sich dabei auf Stegen 4 im Innern des Kopfteils 2 ab, das hohl ausgebildet ist. Gehalten wird die Reibfläche 3 durch eine Art Verrastung mit zueinander korrespondierenden Stegen im Kopfteil 2.

Die Reibfläche 3 ist im dargestellten Ausführungsbeispiel ein von hinten punktförmig geprägtes Schleifblech aus rostfreiem Stahl mit 160 Punkten/cm². Dabei ragen die Prägungen wie Nadelspitzen über die Oberfläche der Reibfläche 3. Statt des geprägten Schleifbleches sind auch andere Reibflächen verwendbar.

Zwischen dem Handgriff 1 und dem Kopfteil 2 ist eine Dreheinrichtung 5 angeordnet. Diese besteht aus einem Zapfen 6, der entsprechend der Abwinkelung des Kopfteils 2 schräg am vorderen Ende des Handgriffs 1 angeformt ist. Dieser Zapfen 6 weist zwei federnde Zungen 7 mit nach außen weisenden Rastnasen 8 an den jeweiligen vorderen Enden auf, wie in der teilweisen Schnittdarstellung in Fig. 1 zu erkennen ist. Das hintere Ende des Kopfteils 2 weist eine dazu korrespondierende Ausnehmung 9 auf. Zum Zusammenfügen des Kopfteils 2 und des Handgriffs 1 wird das Kopfteil 2 mit seiner Ausnehmung 9 auf den zylindrischen Zapfen 6 mit seinen Zungen 7 und Rastnasen 8 gesteckt, wobei die Rastnasen 8 in der Ausnehmung 9 einrasten. Durch den Zapfen 6 wird eine Achse A definiert, um die das Kopfteil 2 drehbar ist, wie durch den Pfeil P angedeutet ist.

Die hintere Stirnseite des Kopfteils 2 weist Vorsprünge 10 auf. Diese korrespondieren mit entsprechenden Ausnehmungen 11 an der vorderen Stirnseite des Handgriffs 1, so daß nach Verdrehen des Kopfteils 2 in die richtige Lage die Vorsprünge 10 in die Ausnehmungen 11 einrasten und somit eine Verdrehsicherung des Kopfteils 2 bezüglich zum Handgriff 1 bilden.

Durch die abgewinkelte Anordnung des Kopfteils 2 am Handgriff 1 weist die Hornhautreibe eine ergonomisch sehr günstige Form auf. Durch entsprechendes Drehen des Kopfteils 2 kann die Reibfläche 3 entweder an der Innenseite (im stumpfen Winkelbereich) oder an der Außenseite (im überstumpfen Winkelbereich) je nach Bedarf angeordnet sein. Im dargestellten Ausführungsbeispiel in Fig. 1 ist die Reibfläche 3 im stumpfen Winkelbereich und damit an der Innenseite der Hornhautreibe angeordnet. Durch Drehen des Kopfteils 2 um 180° entlang der Achse A weist dann die Reibfläche 3 in dieser Darstellung nach unten.

Bezugszeichenliste

| | |
|---|---|
| 1 | Handgriff |
| 2 | Kopfteil |
| 3 | Reibfläche |
| 4 | Steg |
| 5 | Dreheinrichtung |
| 6 | Zapfen |
| 7 | Zunge |
| 8 | Rastnase |
| 9 | Ausnehmung |
| 10 | Vorsprung |
| 11 | Ausnehmung |
| A | Achse |
| P | Pfeil |

**Patentansprüche**

**1.** Hornhautreibe mit einem Handgriff (1) sowie mit einem eine Reibfläche (3) aufweisenden sowie bezüglich zum Handgriff (1) abgewinkelten Kopfteil (2),

**dadurch gekennzeichnet,**

daß das abgewinkelte Kopfteil (2) drehbar um eine entweder in Längsrichtung des Kopfteils (2) oder in Längsrichtung des Handgriffs (1) verlaufende Achse (A) am Handgriff (1) angeordnet ist.

2. Hornhautreibe nach Anspruch 1, dadurch gekennzeichnet, daß sowohl an der Innenseite (stumpfer Winkelbereich) als auch an der Außenseite (überstumpfer Winkelbereich) des Kopfteils (2) jeweils eine Reibfläche (3) angeordnet ist.

3. Hornhautreibe nach Anspruch 1 oder 2, dadurch gekennzeichnet, daß als Dreheinrichtung (5) ein entsprechend der Abwinkelung schräg angeformter Zapfen (6) mit federnden Zungen (7), die an ihren vorderen Enden Rastnasen (8) aufweisen, sowie eine dazu korrespondierende Ausnehmung (9), in die der Zapfen (6) einrastet, vorgesehen ist.

4. Hornhautreibe nach einem der Ansprüche 1 bis 3, gekennzeichnet durch eine Verdrehsicherung zwischen dem Kopfteil (2) und dem Handgriff (1) in Form einer Rasteinrichtung.

5. Hornhautreibe nach Anspruch 4, dadurch gekennzeichnet, daß als Rasteinrichtung zwischen dem Kopfteil (2) und dem Handgriff (1) miteinander korrespondierende Vorsprünge (10) und Ausnehmungen (11) vorgesehen sind.

6. Hornhautreibe nach einem der Ansprüche 1 bis 5, dadurch gekennzeichnet, daß das Kopfteil (2) eine ovale Querschnittsform aufweist, wobei die Reibfläche(n) (3) an der (den) abgeflachten Umfangsfläche(n) angeordnet ist (sind).

7. Hornhautreibe nach einem der Ansprüche 1 bis 6, dadurch gekennzeichnet, daß die Reibfläche (3) als gewölbte Platte ausgebildet ist.

8. Hornhautreibe nach einem der Ansprüche 1 bis 7, dadurch gekennzeichnet, daß die Reibfläche (3) aus rostfreiem Stahl besteht.

9. Hornhautreibe nach einem der Ansprüche 1 bis 8, dadurch gekennzeichnet, daß die Reibfläche (3) nach Art eines Schleifbleches oder einer Feile ausgebildet ist.

10. Hornhautreibe nach einem der Ansprüche 1 bis 9, dadurch gekennzeichnet, daß die Reibfläche (3) von hinten punktförmig geprägt ist.

11. Hornhautreibe nach Anspruch 10, dadurch gekennzeichnet, daß die Reibfläche (3) 100 bis 200, vorzugsweise 160 Punkte/cm² aufweist.

12. Hornhautreibe nach einem der Ansprüche 1 bis 11, dadurch gekennzeichnet, daß die Reibfläche (3) auswechselbar ist.

**Claims**

1. A callous file with a handle (1) and with a head piece (2) at an angle to the handle (1) and having an abrasive surface (3), characterised in that, the angled head piece (2) is arranged swivellably about an axis (A) on the handle (1) running either longitudinally to the head piece (2) or longitudinally to the handle (1).

2. A callous file according to claim 1, characterised in that, an abrasive surface is arranged both on the inside (obtuse angle area) and on the outside (reflex angle area) of the head piece (2).

3. A callous file according to claims 1 or 2, characterised in that, a swivel arrangement (5) is provided a gudgeon (6) shaped obliquely in accordance with the angling with spring mounted tongues (7), which have locking lugs (8) at their front ends, and a corresponding recess (9) in which the gudgeon (6) engages.

4. A callous file according to any one of claims 1 to 3, characterised by means of a twist lock arrangement between the head piece (2) and the handle (1) in the form of a catch arrangement.

5. A callous file according to claim 4, characterised in that, the catch arrangement between the head piece (2) and the handle (1), corresponding projections (10) and recesses (11) are provided.

6. A callous file according to any one of claims 1 to 5, characterised in that, the head piece (2) has an oval cross section, with the abrasive surface(s) (3) arranged on the flattened peripheral surfaces(s).

7. A callous file according to any one of claims 1 to 6, characterised in that, the abrasive surface (3) is shaped as a convex plate.

8. A callous file according to any one of claims 1 to 7, characterised in that, the abrasive surface (3) consists of stainless steel.

9. A callous file according to any one of claims 1 to 8, characterised in that, the abrasive surface (3) has a surface like a rasp or file.

10. A callous file according to any one of claims 1 to 9, characterised in that, the abrasive surface (3) is formed by punching from the back to form pointed shapes.

11. A callous file according to claim 10, characterised in that, the abrasive surface has 100 to 200, though preferably 160 points/cm .

12. A callous file according to any one of claims 1 to 11, characterised in that, the abrasive surface (3) is replaceable.

**Revendications**

1. Râpe pour callosité avec un manche (l) et une partie principale (2) présentant une surface abrasive (3) étant inclinée par rapport au manche (1)
   caractérisé en ce que
   la partie principale inclinée (2) est disposée sur le manche (1) de façon à pouvoir tourner autour d'un axe (A) s'étendant soit en direction longitudinale dè la partie principale (2) soit en direction longitudinale du manche (1).

2. Râpe pour callosité selon la revendication 1, caractérisée en ce que une surface abrasive (3) est disposée aussi bien sur le côté intérieur (zone de l'angle rentrant) que sur le côté extérieur (zone de l'angle saillant) de la partie principale (2).

3. Râpe pour callosité selon l'une quelconque des revendications 1 ou 2, caractérisée en ce qu'il est prévu un tenon (6) en tant que dispositif de rotation (5) formé en biais en fonction de l'inclinaison, avec des languettes (7) faisant ressort, comportant des crans d'arrêt (8) à leurs extrémités antérieures ainsi que la partie creuse (9) correspondante, dans laquelle les crans (6) s'enclenchent.

4. Râpe pour callosité selon l'une quelconque des revendications 1 à 3, caractérisée par un dispositif de blocage entre la partie principale (2) et le manche (1) en forme d'un dispositif à cran d'arrêt.

5. Râpe pour callosité selon la revendication 4, caractérisée en ce que des saillies (10) et des parties creuses (11) correspondant les unes aux autres sont prévues entre la partie principale (2) et le manche (1) constituant un dispositif d'arrêt.

6. Râpe pour callosité selon l'une quelconque des revendications 1 à 5, caractérisée en ce que la partie principale (2) présente une section transversale ovale, la (les) surface(s) abrasive(s) (3) étant disposée(s) sur la (les) face(s) aplatie(s) de la circonférence.

7. Râpe pour callosité selon l'une quelconque des revendications 1 à 6, caractérisée en ce que la surface abrasive (3) est constituée par une plaque recourbée.

8. Râpe pour callosité selon l'une quelconque des revendications 1 à 8, caractérisée en ce que la surface abrasive (3) est en acier inoxydable.

9. Râpe pour callosité selon l'une quelconque des revendications 1 à 7, caractérisée en ce que la surface abrasive (3) est formée comme une tôle de polissage ou une lime.

10. Râpe pour callosité selon l'une quelconque des revendications 1 à 9 caractérisée en ce que la surface abrasive (3) est repoussée par poinçonnage.

11. Râpe pour callosité selon la revendication 10 caractérisée en ce que la surface abrasive (3) comporte de 100 à 200, de préférence 160 points par cm$^2$.

12. Râpe pour callosité selon l'une quelconque des revendications 1 à 11, caractérisée en ce que la surface abrasive (3) est interchangeable.

Fig.1

Fig.2

Fig.3

EP 0 269 040 B1